(19) Europäisches Patentamt
European Patent Office
Office européen des brevets

(11) Veröffentlichungsnummer: **0 019 131**
**B1**

(12) # EUROPÄISCHE PATENTSCHRIFT

(45) Veröffentlichungstag der Patentschrift: **22.12.82**

(21) Anmeldenummer: **80102290.6**

(22) Anmeldetag: **28.04.80**

(51) Int. Cl.³: **C 07 D 249/08,** A 01 N 43/64 // (C07C49/173, 49/16, 49/167, 143/68)

(54) **Fluorierte 1-Triazolyl-butan-Derivate, Verfahren zu ihrer Herstellung und ihre Verwendung als Fungizide.**

(30) Priorität: **10.05.79 DE 2918894**

(43) Veröffentlichungstag der Anmeldung: **26.11.80 Patentblatt 80/24**

(45) Bekanntmachung des Hinweises auf die Patenterteilung: **22.12.82 Patentblatt 82/51**

(84) Benannte Vertragsstaaten: **AT BE CH DE FR GB IT LI NL SE**

(56) Entgegenhaltungen:
**EP-A-0 004 303**
**EP-A-0 006 538**
**EP-A-0 009 707**
**DE-A-2 632 603**
**DE-A-2 811 916**

**Die Akte enthält technische Angaben, die nach dem Eingang der Anmeldung eingereicht wurden und die nicht in dieser Patentschrift enthalten sind.**

(73) Patentinhaber: **BAYER AG, Zentralbereich Patente, Marken und Lizenzen, D-5090 Leverkusen 1, Bayerwerk (DE)**

(72) Erfinder: **Krämer, Wolfgang, Dr., Am Eckbusch 39/162, D-5600 Wuppertal-1 (DE)**
Erfinder: **Büchel, Karl Heinz, Dr., Bergerheide 62, D-5600 Wuppertal-1 (DE)**
Erfinder: **Stetter, Joerg, Dr., Gellertweg 4, D-5600 Wuppertal-1 (DE)**
Erfinder: **Frohberger, Paul-Ernst, Dr., Willi-Baumeister-Strasse 5, D-5090 Leverkusen-1 (DE)**
Erfinder: **Brandes, Wilhelm,Dr., Eichendorffstrasse 3, D-5653 Leichlingen-1 (DE)**
Erfinder: **Scheinpflug, Hans Dr., Am Thelenhof 15, D-5090 Leverkusen-1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

# Fluorierte 1-Triazolylbutanderivate, Verfahren zu ihrer Herstellung und ihre Verwendung als Fungizide

Die Erfindung betrifft neue fluorierte 1-Triazolylbutanderivate, ein Verfahren zu ihrer Herstellung und ihre Verwendung als Fungizide.

Es ist bereits bekannt geworden, dass chlorierte und bromierte 1-Triazolylbutanderivate gute fungizide Eigenschaften aufweisen (vgl. DE-OS Nr. 2632603). Weiterhin ist bekannt geworden, dass 1-(4-Chlorphenyl)-4,4-dimethyl-2-(1,2,4-triazol-1-yl)-3-pentanon eine gute fungizide Wirksamkeit besitzt (vgl. DE-OS Nr. 2734426).

Die Wirkung dieser Verbindungen ist jedoch, insbesondere bei niedrigen Aufwandmengen und -konzentrationen, nicht immer ganz befriedigend.

Es wurden neue fluorierte 1-Triazolylbutanderivate der allgemeinen Formel I gefunden:

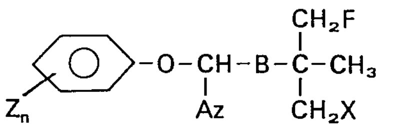 (I)

in welcher:

Az für 1,2,4-Triazol-1-yl oder 1,2,4-Triazol-4-yl,

B für die Ketogruppe oder die CH(OH)-Gruppierung,

X für Wasserstoff oder Fluor,

Z für Halogen, Alkyl mit 1 bis 4 Kohlenstoffatomen, Nitro, Cyano, Alkoxycarbonyl mit 1 bis 4 Kohlenstoffatomen im Alkylteil oder für gegebenenfalls durch Halogen substituiertes Phenyl, und

n für 0, 1, 2 oder 3 stehen,

sowie deren Säure- oder Metallsalzkomplexe.

Diejenigen Verbindungen der allgemeinen Formel I, in welchen B für die CH(OH)-Gruppierung steht, besitzen zwei asymmetrische Kohlenstoffatome; sie können deshalb in den beiden geometrischen Isomeren (threo- und erythro-Form) vorliegen, die in unterschiedlichen Mengenverhältnissen anfallen können. In beiden Fällen liegen sie als optische Isomeren vor. Sämtliche Isomeren werden erfindungsgemäss beansprucht.

Weiterhin wurde gefunden, dass man die fluorierten 1-Triazolylbutanderivate der allgemeinen Formel I erhält, wenn man Halogenetherketone der allgemeinen Formel II

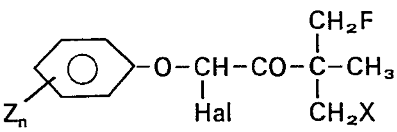 (II)

in welcher

X, Z und n die oben angegebene Bedeutung haben, und

Hal für Halogen, vorzugsweise Chlor oder Brom, steht,

mit 1,2,4-Triazol in Gegenwart eines Säurebindemittels und gegebenenfalls in Gegenwart eines Verdünnungsmittels umsetzt, und gegebenenfalls noch die so erhaltenen Ketoderivate nach bekannten Methoden in üblicher Weise reduziert.

An die so erhaltenen Verbindungen der allgemeinen Formel I können gegebenenfalls anschliessend eine Säure oder ein Metallsalz addiert werden.

Die neuen fluorierten 1-Triazolylbutanderivate weisen starke fungizide Eigenschaften auf. Dabei zeigen überraschenderweise die erfindungsgemässen Verbindungen eine erheblich höhere Wirkung als die aus dem Stand der Technik bekannten chlorierten und bromierten 1-Triazolylbutanderivate, die chemisch und wirkungsmässig naheliegendste Verbindungen sind; und als das ebenfalls bekannte 1-(4-Chlorphenyl)-4,4-dimethyl-2-(1,2,4-triazol-1-yl)-3-pentanon, welches auch chemisch und wirkungsmässig eine naheliegende Verbindung ist. Die erfindungsgemässen Stoffe stellen somit eine Bereicherung der Technik dar.

Die erfindungsgemässen fluorierten 1-Triazolylbutanderivate sind durch die allgemeine Formel I definiert.

Bevorzugt sind diejenigen fluorierten 1-Triazolylbutanderivate, in denen Z für Halogen, Methyl, Ethyl, Nitro, Cyano, Methoxycarbonyl, Ethoxycarbonyl, Phenyl oder Chlorphenyl steht, und der Index n für 0, 1 oder 2 steht.

Im einzelnen seien ausser den bei den Herstellungsbeispielen genannten Verbindungen die folgenden Verbindungen der allgemeinen Formel I genannt:

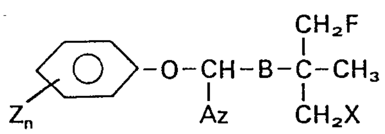 (I)

| $Z_n$ | X | B | Az |
|---|---|---|---|
| - | H | CO | 1,2,4-Triazol-1-yl |
| 2-F | H | CO | 1,2,4-Triazol-1-yl |
| 3-F | H | CO | 1,2,4-Triazol-1-yl |
| 4-F | H | CO | 1,2,4-Triazol-1-yl |
| 2-Cl | H | CO | 1,2,4-Triazol-1-yl |
| 3-Cl | H | CO | 1,2,4-Triazol-1-yl |
| 2-Br | H | CO | 1,2,4-Triazol-1-yl |
| 3-Br | H | CO | 1,2,4-Triazol-1-yl |
| 2-$CH_3$ | H | CO | 1,2,4-Triazol-1-yl |
| 4-$CH_3$ | H | CO | 1,2,4-Triazol-1-yl |
| 2-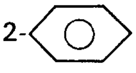 | H | CO | 1,2,4-Triazol-1-yl |
| 4-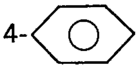 | H | CO | 1,2,4-Triazol-1-yl |
| 4-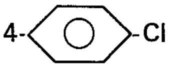 | H | CO | 1,2,4-Triazol-1-yl |
| 2-$NO_2$ | H | CO | 1,2,4-Triazol-1-yl |
| 4-CN | H | CO | 1,2,4-Triazol-1-yl |
| 4-$COOCH_3$ | H | CO | 1,2,4-Triazol-1-yl |
| 4-$COOC_2H_5$ | H | CO | 1,2,4-Triazol-1-yl |
| 4-J | H | CO | 1,2,4-Triazol-1-yl |
| 4-Cl, 2-$CH_3$ | H | CO | 1,2,4-Triazol-1-yl |
| 4-$CH_3$, 2-Cl | H | CO | 1,2,4-Triazol-1-yl |

| $Z_n$ | X | B | Az |
|---|---|---|---|
| - | H | CH(OH) | 1,2,4-Triazol-1-yl |
| 2-F | H | CH(OH) | 1,2,4-Triazol-1-yl |
| 3-F | H | CH(OH) | 1,2,4-Triazol-1-yl |
| 4-F | H | CH(OH) | 1,2,4-Triazol-1-yl |
| 2-Cl | H | CH(OH) | 1,2,4-Triazol-1-yl |
| 3-Cl | H | CH(OH) | 1,2,4-Triazol-1-yl |
| 2-Br | H | CH(OH) | 1,2,4-Triazol-1-yl |
| 3-Br | H | CH(OH) | 1,2,4-Triazol-1-yl |
| 2-$CH_3$ | H | CH(OH) | 1,2,4-Triazol-1-yl |
| 4-$Ch_3$ | H | CH(OH) | 1,2,4-Triazol-1-yl |
| 2-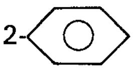 | H | CH(OH) | 1,2,4-Triazol-1-yl |
| 4-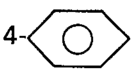 | H | CH(OH) | 1,2,4-Triazol-1-yl |
| 4-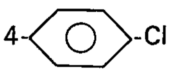 | H | CH(OH) | 1,2,4-Triazol-1-yl |
| 2-$NO_2$ | H | CH(OH) | 1,2,4-Triazol-1-yl |
| 4-CN | H | CH(OH) | 1,2,4-Triazol-1-yl |
| 4-$COOCH_3$ | H | CH(OH) | 1,2,4-Triazol-1-yl |
| 4-$COOC_2H_3$ | H | CH(OH) | 1,2,4-Triazol-1-yl |
| 4-J | H | CH(OH) | 1,2,4-Triazol-1-yl |
| 4-Cl, 2-$CH_3$ | H | CH(OH) | 1,2,4-Triazol-1-yl |
| 4-$CH_3$, 2-Cl | H | CH(OH) | 1,2,4-Triazol-1-yl |

| $Z_n$ | X | B | Az |
|---|---|---|---|
| - | F | CO | 1,2,4-Triazol-1-yl |
| 2-F | F | CO | 1,2,4-Triazol-1-yl |
| 3-F | F | CO | 1,2,4-Triazol-1-yl |
| 3-Cl | F | CO | 1,2,4-Triazol-1-yl |
| 2-Br | F | CO | 1,2,4-Triazol-1-yl |
| 3-Br | F | CO | 1,2,4-Triazol-1-yl |
| 4-Br | F | CO | 1,2,4-Triazol-1-yl |
| 2-$CH_3$ | F | CO | 1,2,4-Triazol-1-yl |
| 4-$CH_3$ | F | CO | 1,2,4-Triazol-1-yl |
| 2-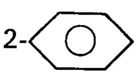 | F | CO | 1,2,4-Triazol-1-yl |
| 4-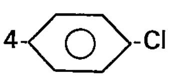 | F | CO | 1,2,4-Triazol-1-yl |
| 2-$NO_2$ | F | CO | 1,2,4-Triazol-1-yl |
| 4-$NO_2$ | F | CO | 1,2,4-Triazol-1-yl |
| 4-CN | F | CO | 1,2,4-Triazol-1-yl |
| 4-$COOCH_3$ | F | CO | 1,2,4-Triazol-1-yl |
| 4-$COOC_2H_5$ | F | CO | 1,2,4-Triazol-1-yl |
| 4-J | F | CO | 1,2,4-Triazol-1-yl |
| 4-Cl, 2-$CH_3$ | F | CO | 1,2,4-Triazol-1-yl |

| $Z_n$ | X | B | Az |
|---|---|---|---|
| - | F | CH(OH) | 1,2,4-Triazol-1-yl |
| 2-F | F | CH(OH) | 1,2,4-Triazol-1-yl |
| 3-F | F | CH(OH) | 1,2,4-Triazol-1-yl |
| 3-Cl | F | CH(OH) | 1,2,4-Triazol-1-yl |
| 2-Br | F | CH(OH) | 1,2,4-Triazol-1-yl |
| 3-Br | F | CH(OH) | 1,2,4-Triazol-1-yl |
| 4-Br | F | CH(OH) | 1,2,4-Triazol-1-yl |

| $Z_n$ | X | B | Az |
|---|---|---|---|
| 2-$CH_3$ | F | CH(OH) | 1,2,4-Triazol-1-yl |
| 4-$CH_3$ | F | CH(OH) | 1,2,4-Triazol-1-yl |
| 2-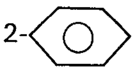 | F | CH(OH) | 1,2,4-Triazol-1-yl |
| 4-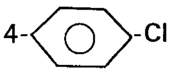 | F | CH(OH) | 1,2,4-Triazol-1-yl |
| 2-$NO_2$ | F | CH(OH) | 1,2,4-Triazol-1-yl |
| 4-$NO_2$ | F | CH(OH) | 1,2,4-Triazol-1-yl |
| 4-CN | F | CH(OH) | 1,2,4-Triazol-1-yl |
| 4-$COOCH_3$ | F | CH(OH) | 1,2,4-Triazol-1-yl |
| 4-$COOC_2H_3$ | F | CH(OH) | 1,2,4-Triazol-1-yl |
| 4-J | F | CH(OH) | 1,2,4-Triazol-1-yl |
| 4-Cl, 2-$CH_3$ | F | CH(OH) | 1,2,4-Triazol-1-yl |
| 4-$CH_3$, 2-Cl | F | CH(OH) | 1,2,4-Triazol-1-yl |

Verwendet man beispielsweise 1-Brom-1-(4-chlorphenoxy)-3,3-dimethyl-4-fluorbutan-2-on und 1,2,4-Triazol als Ausgangsstoffe, so kann der Reaktionsablauf durch das folgende Formelschema wiedergegeben werden:

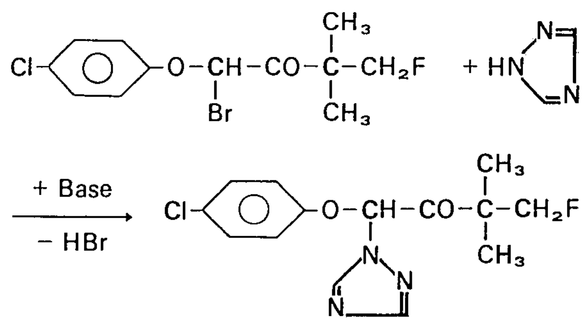


Verwendet man 1-(4-Chlorphenoxy)-3,3-dimethyl-4-fluor-1-(1,2,4-triazol-1-yl)butan-2-on und Natriumborhydrid als Ausgangsstoffe, so kann der Reaktionsablauf der Reduktion durch das folgende Formelschema wiedergegeben werden:

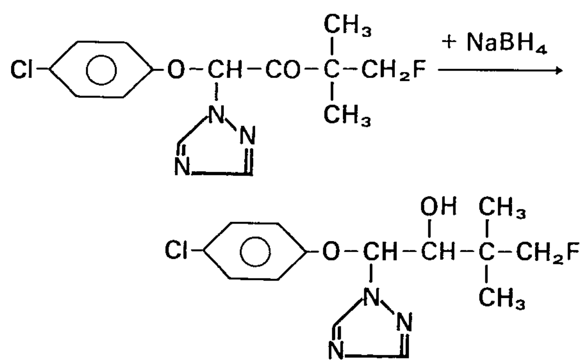


Die bei der Durchführung des erfindungsgemässen Verfahrens als Ausgangsstoffe zu verwendenden Halogenetherketone sind durch die allgemeine Formel II definiert. In dieser Formel stehen X, Z und der Index n vorzugsweise für diejenigen Reste, die bereits im Zusammenhang mit der Beschreibung der erfindungsgemässen Stoffe der allgemeinen Formel I vorzugsweise für diese Substituenten genannt wurden.

Die Halogenetherketone der allgemeinen Formel II sind noch nicht bekannt. Sie können jedoch nach bekannten Verfahren (vgl. z.B. DE-OS Nr. 2632603) erhalten werden, indem man z.B. bekannte Phenole der allgemeinen Formel III

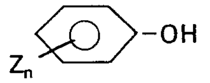

(III)

in welcher

Z und n die oben angegebene Bedeutung haben,

mit einem Halogenketon der allgemeinen Formel IV

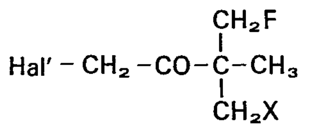

(IV)

in welcher

X die oben angegebene Bedeutung hat, und

Hal' für Chlor oder Brom steht,

umsetzt. Das noch verbliebene aktive Wasserstoffatom wird anschliessend in üblicher Weise gegen ein Halogenatom ausgetauscht (vgl. auch die Herstellungsbeispiele).

Die Halogenketone der allgemeinen Formel IV sind ebenfalls noch nicht bekannt. Sie können jedoch auf allgemein übliche und bekannte Weise erhalten werden, indem man Fluorderivate des 3,3-Dimethylbutan-2-ons der allgemeinen Formel V

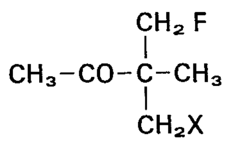

(V)

in welcher

X die oben angegebene Bedeutung hat,

mit Chlor oder Brom in Gegenwart eines inerten organischen Lösungsmittels, wie beispielsweise Ether oder chlorierte Kohlenwasserstoffe, bei Raumtemperatur versetzt (vgl. auch die Herstellungsbeispiele), oder mit üblichen Chlorierungsmitteln, wie beispielsweise Sulfurylchlorid bei 20 bis 60° C umsetzt.

Die Fluorderivate des 3,3-Dimethylbutan-2-ons der allgemeinen Formel V sind Gegenstand einer eigenen älteren Patentanmeldung (vgl. DE-OS Nr. 2843767). Man erhält die Verbindungen der allgemeinen Formel V, wenn man Sulfonsäureester der allgemeinen Formel VI

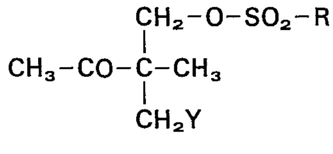

(VI)

in welcher

R für Alkyl mit 1 bis 4 Kohlenstoffatomen, insbesondere Methyl, oder Aryl mit 6 bis 12 Kohlenstoffatomen, insbesondere Phenyl oder Tolyl, steht, und

Y für Wasserstoff oder die Gruppe $-O-SO_2-R$ steht,

mit Metallfluoriden, wie beispielsweise Natrium- und Kaliumfluorid, in Gegenwart eines polaren organischen Lösungsmittels, wie beispielsweise Di-, Tri- oder Tetraethylenglykol, bei Temperaturen zwischen 80 und 250° C umsetzt (vgl. auch die Herstellungsbeispiele).

Die Sulfonsäureester der allgemeinen Formel VI sind teilweise bekannt [„J. Org. Chem." *35*, 2391 (1970)]. Die noch nicht beschriebenen Verbindungen können nach literaturbekannten Verfahren aus den entsprechenden Hydroxybutanonen und Sulfochloriden in Gegenwart von Basen hergestellt werden (z.B. Houben-Weyl, „Methoden der Org. Chemie", Bd. IX, S. 388 u. 663, sowie die Angaben bei den Herstellungsbeispielen).

Für die erfindungsgemässe Umsetzung kommen als Verdünnungsmittel inerte organische Lösungsmittel in Frage. Hierzu gehören vorzugsweise Ketone, wie Diethylketon und insbesondere Aceton und Methylethylketon; Nitrile, wie Propionitril, insbesondere Acetonitril; Alkohole, wie Ethanol oder Isopropanol; Ether, wie Tetrahydrofuran oder Dioxan; Benzol; Toluol; Formamide, wie insbesondere Dimethylformamid; und halogenierte Kohlenwasserstoffe.

Die erfindungsgemässe Umsetzung wird in Gegenwart eines Säurebinders vorgenommen. Man kann alle üblicherweise verwendbaren anorganischen oder organischen Säurebinder zugeben, wie Alkalicarbonate, beispielsweise Natriumcarbonat, Kaliumcarbonat und Natriumhydrogencarbonat, oder wie niedere tertiäre Alkylamine, Cycloalkylamine oder Aralkylamine, beispielsweise Triethylamin, N,N-Dimethylcyclohexylamin, Dicyclohexylamin, N,N-Dimethylbenzylamin, weiterhin Pyridin und Diazabicyclooctan.

Vorzugsweise verwendet man einen entsprechenden Überschuss an Triazol.

Die Reaktionstemperaturen können in einem grösseren Bereich variiert werden. Im allgemeinen arbeitet man zwischen etwa 20 bis etwa 150° C, vorzugsweise bei 60 bis 120° C. Bei Anwesenheit eines Lösungsmittels wird zweckmässigerweise beim Siedepunkt des jeweiligen Lösungsmittels gearbeitet.

Bei der Durchführung des erfindungsgemässen Verfahrens setzt man auf 1 mol der Verbindungen der allgemeinen Formel II vorzugsweise 2 mol Triazol und 1 bis 2 mol Säurebinder ein. Zur Isolierung der Verbindungen der allgemeinen Formel I wird das Lösungsmittel abdestilliert, der Rückstand mit einem organischen Solvens aufgenommen und mit Wasser gewaschen. Die organische Phase wird über Natriumsulfat getrocknet und im Vakuum vom Lösungsmittel befreit. Der Rückstand wird durch Destillation bzw. Umkristallisation oder Salzbildung und Umkristallisation gereinigt.

Die erfindungsgemässe Reduktion erfolgt in üblicher Weise, wie z.B. durch Umsetzung mit komplexen Hydriden, gegebenenfalls in Gegenwart eines Verdünnungsmittels, oder durch Umsetzung mit Aluminiumisopropylat in Gegenwart eines Verdünnungsmittels.

Arbeitet man mit komplexen Hydriden, so kommen als Verdünnungsmittel für die erfindungsgemässe Umsetzung polare organische Lösungsmittel in Frage. Hierzu gehören vorzugsweise Alkohole, wie Methanol, Ethanol, Butanol, Isopropanol, und Ether, wie Diethylether oder Tetrahydrofuran. Die Reaktion wird im allgemeinen bei 0 bis 30° C, vorzugsweise bei 0 bis 20° C durchgeführt. Hierzu setzt man auf 1 mol des Ketons der allgemeinen Formel I etwa 1 mol eines komplexen Hydrids, wie Natriumborhydrid oder Lithiumalanat, ein. Zur Isolierung der reduzierten Verbindungen der allgemeinen Formel I wird der Rückstand in verdünnter Salzsäure aufgenommen, anschliessend alkalisch gestellt und mit einem organischen Lösungsmittel extrahiert. Die weitere Aufarbeitung erfolgt in üblicher Weise.

Arbeitet man mit Aluminiumisopropylat, so kommen als Verdünnungsmittel für die erfindungsgemässe Umsetzung bevorzugt Alkohole, wie Isopropanol, oder inerte Kohlenwasserstoffe, wie Benzol, in Frage. Die Reaktionstemperaturen können wiederum in einem grösseren Bereich variiert werden; im allgemeinen arbeitet man zwischen 20 und 120° C, vorzugsweise bei 50 bis 100° C. Zur Durchführung der Reaktion setzt man auf 1 mol des Ketons der allgemeinen Formel I etwa 0,3 bis 2 mol Aluminiumisopropylat ein. Zur Isolierung der reduzierten Verbindungen der allgemeinen Formel I wird das überschüssige Lösungsmittel im Vakuum entfernt und die entstandenen Aluminiumverbindungen mit verdünnter Schwefelsäure oder Natronlauge zersetzt. Die weitere Aufarbeitung erfolgt in üblicher Weise.

Zur Herstellung von Säureadditionssalzen der Verbindungen der allgemeinen Formel I kommen alle physiologisch verträglichen Säuren in Frage. Hierzu gehören vorzugsweise die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z.B. p-Toluolsulfonsäure und 1,5-Naphthalindisulfonsäure.

Die Salze der Verbindungen der allgemeinen Formel I können in einfacher Weise nach üblichen Salzbildungsmethoden, z.B. durch Lösen einer Verbindung der allgemeinen Formel I in einem geeigneten inerten Lösungsmittel, und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel oder durch Umkristallisation gereinigt werden.

Zur Herstellung von Metallsalzkomplexen der Verbindungen der allgemeinen Formel I kommen vorzugsweise Salze von Metallen der II. bis IV. Haupt- und der I. und II. sowie IV. bis VIII. Nebengruppe in Frage, wobei Kupfer, Zink, Mangen, Magnesium, Zinn, Eisen und Nickel beispielhaft genannt seien. Als Anionen der Salze kommen solche in Betracht, die sich von physiologischen Säuren ableiten. Hierzu gehören vorzugsweise die Halogenwasserstoffsäuren, wie z.B. die Chlorwasserstoffsäure und die Bromwasserstoffsäure, ferner Phosphorsäure, Salpetersäure und Schwefelsäure.

Die Metallsalzkomplexe der Verbindungen der allgemeinen Formel I können in einfacher Weise nach üblichen Verfahren erhalten werden, so z.B. durch Lösen des Metallsalzes in Alkohol, z.B. Äthanol, und Hinzufügen zur Verbindung der allgemeinen Formel I. Man kann Metallsalzkomplexe in bekannter Weise, z.B. durch Abfiltrieren, Isolieren und gegebenenfalls durch Umkristallisieren, reinigen.

Die erfindungsgemässen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes und Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemässen Wirkstoffe mit besonders gutem Erfolg zur Bekämpfung des Gurkenmehltaus *(Erysiphe cichoracearum)* sowie gegen Getreidekrankheiten, wie gegen Getreidemehltau, Getreiderost und Gerstenmehltau eingesetzt werden. Hervorzuheben ist, dass die erfindungsgemässen Wirkstoffe nicht nur eine protektive Wirkung entfalten, sondern auch systemisch wirksam sind. So gelingt es, Pflanzen gegen Pilzbefall zu schützen, wenn man den Wirkstoff über den Boden und die Wurzel, oder über das Saatgut, den oberirdischen Teilen der Pflanze zuführt.

Die Wirkstoffe können in die üblichen Formulierungen übergeführt werden, wie Lösungen, Emulsionen, Suspensionen, Pulver, Schäume, Pasten, Granulate, Aerosole, wirkstoffimprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebelformulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder

chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosoltreibgas, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengel; als Emulgier- und/oder schaumerzeugende Mittel kommen in Frage: z.B. nichtionogene und anionische Emulgatoren, wie Polyoxyäthylenfettsäureester, Polyoxyäthylenfettalkoholäther, z.B. Alkylarylpolyglykoläther, Alkylsulfonate, Alkylsulfate, Arylsulfonate sowie Eiweisshydrolysate; als Dispergiermittel kommen in Frage: z.B. Ligninsulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azolmetallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.% Wirkstoff, vorzugsweise zwischen 0,5 und 90%.

Die erfindungsgemässen Wirkstoffe können in den Formulierungen oder in den verschiedenen Anwendungsformen in Mischung mit anderen bekannkten Wirkstoffen vorliegen, wie Fungiziden, Bakteriziden, Insektiziden, Akariziden, Nematiziden, Herbiziden, Schutzstoffen gegen Vogelfrass, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Giessen, Tauchen, Spritzen, Sprühen, Vernebeln, Verdampfen, Injizieren, Verschlämmen, Verstreichen, Stäuben, Streuen, Trockenbeizen, Feuchtbeizen, Nassbeizen, Schlämmbeizen oder Inkrustieren.

Bei der Behandlung von Pflanzenteilen können die Wirkstoffkonzentrationen in den Anwendungsformen in einem grösseren Bereich variiert werden. Sie liegen im allgemeinen zwischen 1 und 0,0001 Gew.%, vorzugsweise zwischen 0,5 und 0,001%.

Bei der Saatgutbehandlung werden im allgemeinen Wirkstoffmengen von 0,001 bis 50 g je kg Saatgut, vorzugsweise 0,01 bis 10 g, benötigt.

Bei Behandlung des Bodens sind Wirkstoffkonzentrationen von 0,00001 bis 0,1 Gew.%, vorzugsweise von 0,0001 bis 0,02%, am Wirkungsort erforderlich.

In den nachfolgenden Beispielen werden die nachstehend angegebenen Verbindungen als Vergleichssubstanzen eingesetzt:

(A) = 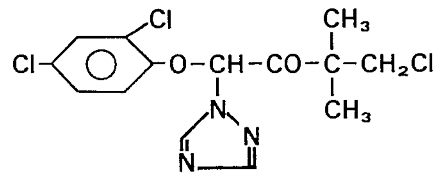

(B) = 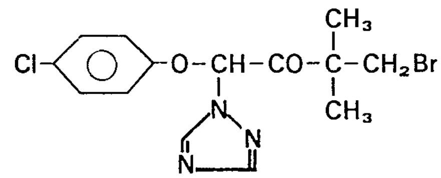 × HCl

(C) = 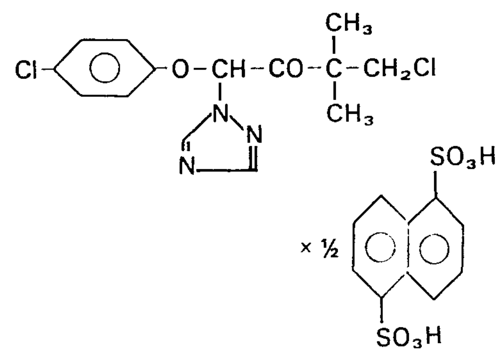

× ½

(D) = 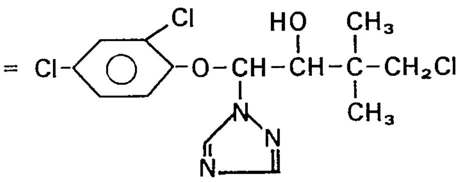

(E) = 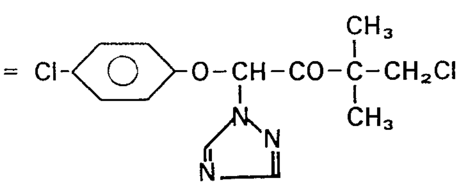

(F) = 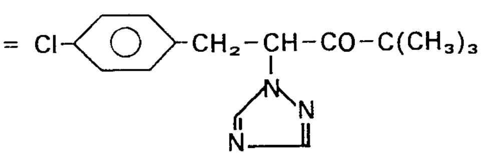

*Beispiel A*

*Sprossbehandlungstest / Getreidemehltau / protektiv (blattzerstörende Mykose)*

Zur Herstellung einer zweckmässigen Wirkstoffzubereitung nimmt man 0,25 Gew.-Teile Wirkstoff in 25 Gew.-Teilen Dimethylformamid und 0,06 Gew.-Teilen Emulgator (Alkylarylpolyglykolether) auf und gibt 975 Gew.-Teile Wasser hinzu. Das Konzentrat verdünnt man mit Wasser auf die gewünschte Endkonzentration der Spritzbrühe.

Zur Prüfung auf protektive Wirksamkeit besprüht man die einblättrigen Gerstenjungpflanzen der Sorte-Amsel mit der Wirkstoffzubereitung taufeucht. Nach Antrocknen bestäubt man die Gerstenpflanzen mit Sporen von *Erysiphe graminis* var. *hordei.*

Nach 6 d Verweilzeit der Pflanzen bei einer Temperatur von 21-22° C und einer Luftfeuchtigkeit von 80-90% wertet man den Besatz der Pflanzen mit Mehltaupusteln aus. Der Befallsgrad wird in Prozent des Befalls der unbehandelten Kontrollpflanzen ausgedrückt. Dabei bedeutet 0% keinen Befall und 100% den gleichen Befallsgrad wie bei der unbehandelten Kontrolle. Der Wirkstoff ist um so wirksamer, je geringer der Mehltaubefall ist.

Bei diesem Test zeigen z.B. die folgenden Verbindungen eine sehr gute Wirkung, die derjenigen der aus dem Stand der Technik bekannten Verbindungen A, B, C und D überlegen ist: Verbindungen gemäss Herstellungsbeispielen 1, 2, 3, 4, 5, 6, 7, 8, 9, 10, 14, 15, 17 und 18.

*Beispiel B*

*Gerstenmehltautest* (Erysiphe graminis *var.* hordei) */ systemisch (pilzliche Getreidesprosskrankheit)*

Die Anwendung der Wirkstoffe erfolgt als pulverförmige Saatgutbehandlungsmittel. Sie werden hergestellt durch Abstrecken des Wirkstoffes mit einem Gemisch aus gleichen Gew.-Teilen Talkum und Kieselgur zu einer feinpulverigen Mischung mit der gewünschten Wirkstoffkonzentration.

Zur Saatgutbehandlung schüttelt man Gerstensaatgut mit dem abgestreckten Wirkstoff in einer verschlossenen Glasflasche. Das Saatgut sät man mit 3×12 Korn in Blumentöpfe 2 cm tief in ein Gemisch aus einem Volumenteil Frühstorfer Einheitserde und einem Volumenteil Quarzsand ein. Die Keimung und der Auflauf erfolgen unter günstigen Bedingungen im Gewächshaus. 7 d nach der Aussaat, wenn die Gerstenpflanzen ihr erstes Blatt entfaltet haben, werden sie mit frischen Sporen von *Erysiphe graminis* var. *hordei* bestäubt und bei 21-22° C und 80-90% rel. Luftfeuchte und 16stündiger Belichtung weiter kultiviert. Innerhalb von 6 d bilden sich an den Blättern die typischen Mehltaupusteln aus.

Der Befallsgrad wird in Prozent des Befalls der unbehandelten Kontrollpflanzen ausgedrückt. So bedeutet 0% keinen Befall und 100% den gleichen Befallsgrad wie bei der unbehandelten Kontrolle. Der Wirkstoff ist um so wirksamer, je geringer der Mehltaubefall ist.

Bei diesem Test zeigen z.B. die folgenden Verbindungen eine sehr gute Wirkung, die derjenigen der aus dem Stand der Technik bekannten Verbindungen B, C und E überlegen ist: Verbindungen gemäss Herstellungsbeispielen: 1, 2, 3, 4, 5, 6, 7, 10, 14, 15 und 17.

*Beispiel C*

*Erysiphetest (Gurken) / protektiv*

| | |
|---|---|
| Lösungsmittel: | 4,7 Gew.-Teile Aceton |
| Emulgator: | 0,3 Gew.-Teile Alkylarylpolyglykolether |
| Wasser: | 95,0 Gew.-Teile |

Man vermischt die für die gewünschte Wirkstoffkonzentration in der Spritzflüssigkeit nötige Wirkstoffmenge mit der angegebenen Menge des Lösungsmittels und verdünnt das Konzentrat mit der angegebenen Menge Wasser, welches die genannten Zusätze enthält.

Mit der Spritzflüssigkeit bespritzt man junge Gurkenpflanzen mit etwa drei Laubblättern bis zur Tropfnässe. Die Gurkenpflanzen verbleiben zur Trocknung 24 h im Gewächshaus. Dann werden sie zur Inokulation mit Konidien des Pilzes *Erysiphe cichoracearum* bestäubt. Die Pflanzen werden anschliessend bei 23 bis 24° C und bei einer rel. Luftfeuchtigkeit von ca. 75% im Gewächshaus aufgestellt.

Nach 12 d wird der Befall der Gurkenpflanzen bestimmt. Die erhaltenen Boniturwerte werden in Prozent Befall umgerechnet. 0% bedeutet keinen Befall, 100% bedeutet, dass die Pflanzen vollständig befallen sind.

Bei diesem Test zeigen z.B. die folgenden Verbindungen eine sehr gute Wirkung, die derjenigen der aus dem Stand der Technik bekannten Verbindung F überlegen ist: Verbindungen gemäss Herstellungsbeispielen: 1, 2, 3, 4, 5, 6, 7, 8, 14, 15, 16 und 17.

*Herstellungsbeispiele*

*Beispiel 1*

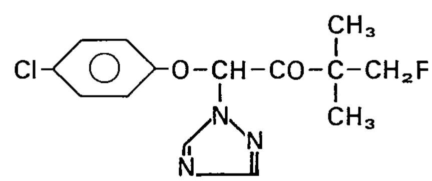

87 g (0,27 mol) 1-Brom-1-(4-chlorphenoxy)-3,3-dimethyl-4-fluor-2-butanon werden in 200 ml Aceton gelöst und zu einer siedenden Lösung von 46 g (0,66 mol) 1,2,4-Triazol in 200 ml Aceton getropft. Nach 1 h Erhitzen unter Rückfluss wird das Lösungsmittel im Vakuum entfernt, der Rückstand mit Methylenchlorid aufgenommen, mehrmals mit Wasser gewaschen und die organische Phase über Natriumsulfat getrocknet. Nach Entfernen des Lösungsmittels im Vakuum wird der Röckstand mit Petrolether kristallisiert.

Man erhält 75 g (89% der Theorie) 1-(4-Chlorphenoxy)-3,3-dimethyl-4-fluor-1-(1,2,4-triazol-1-yl)-2-butanon vom Schmelzpunkt 60-63° C.

*Herstellung der Vorstufen*

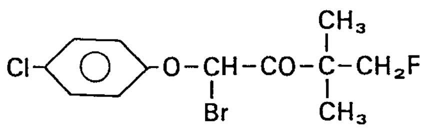

175 g (0,71 mol) 1-(4-Chlorphenoxy)-3,3-dimethyl-4-fluor-2-butanon werden in 500 ml Methylenchlorid gelöst und bei 20 bis 30° C unter Rühren und Kühlen tropfenweise mit 114 g (0,71 mol) Brom versetzt. Es wird 2 h bei 20° C nachgerührt, vorsichtig mit 200 ml Wasser versetzt, die Methylenchloridphase mehrmals mit Eiswasser gewaschen und über Natriumsulfat getrocknet.

Nach Abdestillieren des Lösungsmittels im Vakuum wird der Rückstand aus Cyclohexan umkristallisiert. Man erhält 180 g (78% der Theorie) 1-Brom-1-(4-chlorphenoxy)-3,3-dimethyl-4-fluor-2-butanon vom Schmelzpunkt 73-75° C.

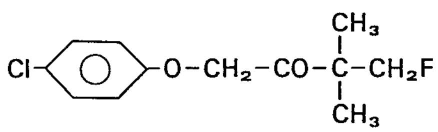

Zu einer gerührten Mischung aus 102 g (0,79 mol) p-Chlorphenol und 110 g (0,79 mol) gepulvertem Kaliumcarbonat in 500 ml Aceton werden unter Kühlen bei 20 bis 30° C 157 g (0,79 mol) 1-Brom-3,3-dimethyl-4-fluor-2-butanon zugetropft. Es wird 2 h bei 20° C nachgerührt, das anorganische Salz abfiltriert und das Filtrat eingeengt. Der Rückstand wird im Hochvakuum destilliert. Man erhält 175 g (90% der Theorie) 1-(4-Chlorphenoxy)-3,3-dimethyl-4-fluor-2-butanon vom Siedepunkt 112-119° C/0,05 mm Hg-Säule.

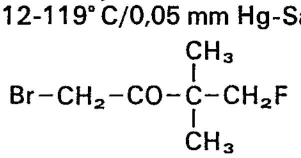

In eine Mischung aus 354 g (3 mol) 3,3-Dimethyl-4-fluor-2-butanon und 2000 ml Ether werden bei 20 bis 30° C unter Kühlen und Rühren 480 g Brom langsam eingetropft. Die gelbliche Lösung wird noch 1 h bei 20° C nachgerührt und anschliessend vorsichtig mit 500 ml Wasser versetzt. Die Etherphase wird abgetrennt, mehrmals mit Wasser gewaschen und über Natriumsulfat getrocknet. Nach Abdestillieren des Lösungsmittels wird der Rückstand im Wasserstrahlvakuum destilliert. Man erhält 472 g (80% der Theorie) 1-Brom-3,3-dimethyl-4-fluor-2-butanon vom Siedepunkt 80-90° C/11 mm Hg-Säule.

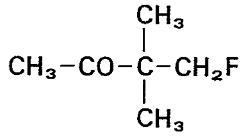

Zu der in einem Dreihalsrührkolben mit absteigendem Kühler befindlichen Suspension von 23,2 g (0,4 mol) trockenem Kaliumfluorid in 400 ml dest. Tetraethylenglykol werden bei 160° C und 20 mbar 38,8 g (0,2 mol) 2,2-Dimethyl-2-oxobutylmethansulfonat im Verlauf von 2 h zugetropft und 2 weitere h nachgerührt. An einem absteigenden Kondensator und in einer nachgeschalteten Tiefkühlfalle wird das herausdestillierte Reaktionsprodukt kondensiert und gesammelt. Man erhält 20,9 g (89% der Theorie) 3,3-Dimethyl-4-fluor-2-butanon vom Siedepunkt 130-134° C.

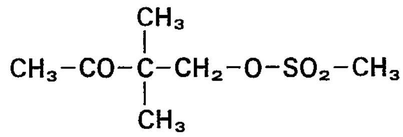

232 g (2 mol) 3,3-Dimethyl-4-hydroxy-2-butanon (zur Herstellung, vgl. Beilstein, *H 1*, E III 3239, IV 4030 und „Bull. Soc. Chim.", France 1964, 2849) werden in 700 ml absolutem Pyridin bei 0 bis 5° C mit 229 g (2 mol) Methansulfochlorid umgesetzt. Nach 12 h Stehen bei 20° C wird mit Methylenchlorid verdünnt und mit Eiswasser ausgeschüttelt. Die organische Phase wird getrocknet, im Vakuum vom Lösungsmittel befreit und über eine Kolonne fraktioniert. Man erhält 332 g (86% der Theorie) 2,2-Dimethyl-3-oxobutylmethansulfonat vom Siedepunkt 106-120° C/0,12 mm Hg-Säule.

*Beispiel 2*

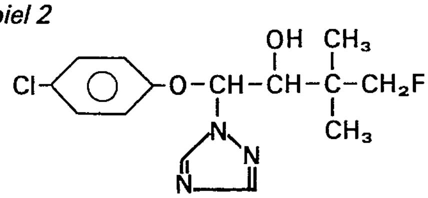

55 g (0,176 mol) 1-(4-Chlorphenoxy)-4-fluor-3,3-dimethyl-1-(1,2,4-triazol-1-yl)-2-butanon (Beispiel 1) werden in 250 ml Methanol gelöst und portionsweise mit 3 g (0,08 mol) Natriumborhydrid versetzt. Man lässt die Reaktionslösung 1 h nachrühren und stellt sie dann mit konzentrierter Salzsäure auf einen pH-Wert von 3 ein. Nach Abdestillieren des Lösungsmittels im Vakuum wird der Rückstand mit Wasser versetzt und mit Methylenchlorid ausgeschüttelt. Die vereinigten organischen Phasen werden über Natriumsulfat getrocknet und im Vakuum eingeengt. Der Rückstand wird aus Petrolether umkristallisiert. Man erhält 40 g (72% der Theorie) 1-(4-Chlorphenoxy)-4-fluor-3,3-dimethyl-1-(1,2,4-triazol-1-yl)-2-butanol vom Schmelzpunkt 103-112° C.

*Beispiel 3*

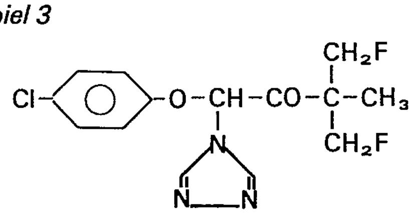

und

*Beispiel 4*

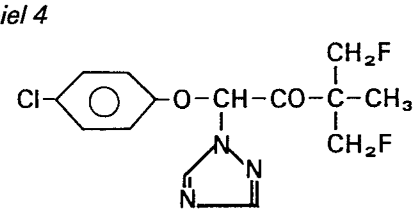

82 g (0,286 mol) 3,3-Bisfluormethyl-1-brom-1-(4-chlorphenoxy)butan-2-on und 41,5 g (0,588 mol) Triazol werden in 600 ml Acetonitril vorgelegt, 5 h bei 50° C erhitzt, das Lösungsmittel im Wasserstrahlvakuum abgetragen, der Rückstand in 1 l Methylenchlorid aufgenommen und zweimal mit je 1000 ml Wasser gewaschen, die organische Phase über Natriumsulfat getrocknet und das Lösungsmittel abdestilliert. Der Rückstand wird in 500 ml Diisopropylether aufgenommen und der Niederschlag abgesaugt. Man erhält 6 g 3,3-Bisfluormethyl-1-(4-chlorphenoxy)-1-(1,2,4-triazol-4-yl)butan-2-on (Beispiel 3) vom Schmelzpunkt 119-122° C. Die Mutterlauge wird destilliert. Man erhält 32,1 g (40% der Theorie) 3,3-Bisfluormethyl-1-(4-chlorphenoxy)-1-(1,2,4-triazol-1-yl)butan-2-on (Beispiel 4) vom Siedepunkt 160-166° C/0,2 mm Hg-Säule.

*Herstellung der Vorstufen*

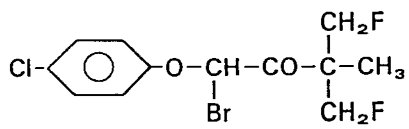

Entsprechend Beispiel 1 durch Umsetzung von 3,3-Bisfluormethyl-1-(4-chlorphenoxy)butan-2-on mit Brom.

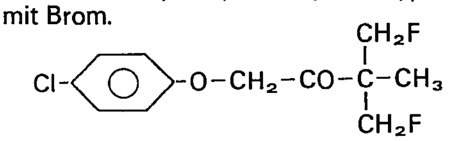

Entsprechend Beispiel 1 durch Umsetzung von p-Chlorphenol mit 3,3-Bisfluormethyl-1-brombutan-2-on.

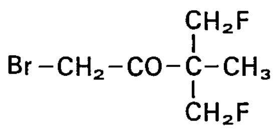

Entsprechend Beispiel 1 durch Umsetzung von 3,3-Bisfluormethylbutan-2-on mit Brom.

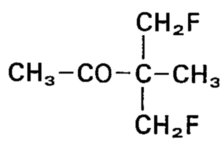

In einem Dreihalskolben mit Rührer, Tropftrichter und Liebigkühler mit gekühlter Vorlage werden 400 ml Tetraethylenglykol und 46,4 g Kaliumfluorid (0,8 mol) vorgelegt und auf 170° C aufgeheizt. Man legt an den Vorstoss des Liebigkühlers ein Wasserstrahlvakuum (Druck ca. 20 bis 30 mbar) an. Dann werden während 45 min 57,6 g (0,2 mol) 2-Acetyl-2-methylpropan-1,3-diolbismethansulfonat, gelöst in 100 ml Tetraethylenglykol, zugetropft. Das entstehende 3,3-Bisfluormethylbutan-2-on wird während der Reaktion in die gekühlte Vorlage abdestilliert. Nach dem Zutropfen wird noch während 1 h bei 175° C weiter destilliert.

Das aufgefangene Destillat wird anschliessend redestilliert. Man erhält 14 g (ca. 51,5% der Theorie) 3,3-Bisfluormethylbutan-2-on vom Siedepunkt 43-46° C/12 mm Hg-Säule.

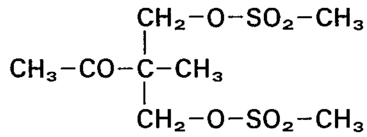

66 g (0,5 mol) 3-Oxa-2,2-bis(hydroxymethyl)-butan (zur Herstellung vgl. Beilstein, *H 1*, E III 3306, IV 4132 und „J. Chem. Soc.", London 1932, 2671) werden in 300 ml 1,2-Dichlorethan gelöst, 114,5 g (1 mol) Methansulfonsäurechlorid zugetropft und bei 0 bis 5° C 158 g (2 mol) Pyridin zugetropft. Man lässt 15 h bei Raumtemperatur nachrühren und gibt dann den Ansatz auf 600 ml Eiswasser und 100 ml konz. Salzsäure. Dabei fällt ein Feststoff aus, der abgesaugt wird. Die wässrige Phase wird mit 1000 ml Methylenchlorid extrahiert; in der Methylenchloridphase wird der Feststoff gelöst, die organische Phase über Natriumsulfat getrocknet, das Lösungsmittel im Wasserstrahlvakuum abdestilliert und der Rückstand in 200 ml Ether suspendiert. Der Rückstand wird abgesaugt und mit 100 ml Ether gewaschen. Man erhält 100 g (ca. 70% der Theorie) 2-Acetyl-2-methylpropan-1,3-diolbismethansulfonat vom Schmelzpunkt 105-108° C.

In entsprechender Weise werden die nachfolgenden Beispiele der allgemeinen Formel I

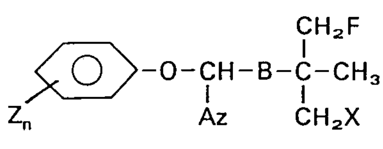 (I)

erhalten:

| Bsp. Nr. | $Z_n$ | B | X | 1,2,4-Triazol-1-yl oder 4-yl | Schmelzpunkt (° C) bzw. Siedepunkt (° C) /Säule (mm Hg) |
|---|---|---|---|---|---|
| 5 | 4-Br | CO | H | 1-yl | 131 (×HCl) |
| 6 | 2,4-$Cl_2$ | CO | H | 1-yl | 213 (Zers.) (×½ NDS) |
| 7 | 4-Cl | CO | H | 1-yl | 260-65 (×½ NDS) |
| 8 | 4- 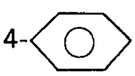 | CO | F | 1-yl | 110 (×½ NDS) |
| 9 | 4-F | CO | F | 1-yl | 144-46/0,2 |

| Bsp. Nr. | $Z_n$ | B | X | 1,2,4-Triazol-1-yl oder 4-yl | Schmelzpunkt (° C) bzw. Siedepunkt (° C) /Säule (mm Hg) |
|---|---|---|---|---|---|
| 10 | 2,4-$Cl_2$ | CO | H | 4-yl | 139 |
| 11 | 4-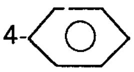 | CO | H | 4-yl | 194 |
| 12 | 4-F | CO | F | 4-yl | 108 |
| 13 | 4-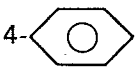 | CO | F | 4-yl | 150 |
| 14 | 2,4-$Cl_2$ | CH(OH) | H | 1-yl | 142-52 |
| 15 | 4-Br | CH(OH) | H | 1-yl | 117-40 |
| 16 | 4-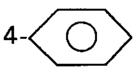 | CH(OH) | F | 1-yl | 120 |
| 17 | 4-Cl | CH(OH) | F | 1-yl | 98 |
| 18 | 4-F | CH(OH) | F | 1-yl | 95-97 |
| 19 | 2-Cl | CO | F | 1-yl | 157-60/0,3 |
| 20 | 2,4-$Cl_2$ | CO | F | 1-yl | 167/0,3 |
| 21 | 2-Cl, 4-$CH_3$ | CO | F | 1-yl | 162/0,3 |
| 22 | 2-Cl | CO | F | 4-yl | 122-26 |
| 23 | 2,4-$Cl_2$ | CO | F | 4-yl | 134-40 |
| 24 | 2-Cl | CH(OH) | F | 1-yl | 96-102 |
| 25 | 2,4-$Cl_2$ | CH(OH) | F | 1-yl | 90-95 |
| 26 | 4-Cl | CH(OH) | F | 1-yl | 124 |
| 27 | 4-F | CH(OH) | F | 1-yl | 89-91 |
| 28 | 2,4-$Cl_2$ | CH(OH) | F | 1-yl | 91-99 |
| 29 | 4-Br | CO | F | 1-yl | 167-70/0,1 |
| 30 | 4-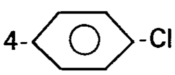-Cl | CO | F | 1-yl | 80-94 |
| 31 | 4-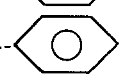 | CO | F | 1-yl | 75-80 |
| 32 | 3-Cl | CO | F | 1-yl | 169-73/0,5 |
| 33 | 4-Br | CH(OH) | F | 1-yl | 140-43 |
| 34 | 4-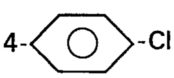-Cl | CH(OH) | F | 1-yl | 165-68 |
| 35 | 3-Cl | CH(OH) | F | 1-yl | 98-104 |
| 36 | 4-Cl, 2-$CH_3$ | CH(OH) | F | 1-yl | 108 |
| 37 | 4-$CH_3$ | CH(OH) | F | 1-yl | 109-11 |
| 38 | 4-$NO_2$ | CO | F | 1-yl | 84-86 |
| 39 | 2-F | CH(OH) | H | 1-yl | 175 (×HCl) |
| 40 | 4-$CH_3$ | CO | F | 1-yl | 150-55/0,1 |
| 41 | 4-$COOCH_3$ | CO | F | 1-yl | Öl |
| 42 | 4-$COOC_2H_5$ | CO | F | 1-yl | 178-80/0,2 |
| 43 | 4-CN | CO | F | 1-yl | Öl |
| 44 | 3-Br | CO | F | 1-yl | 160-63/0,06 |
| 45 | 2-Br | CO | F | 1-yl | 162-65/0,1 |
| 46 | 3-Br | CH(OH) | F | 1-yl | 113 |
| 47 | 3,4-$Cl_2$ | CO | H | 1-yl | 60-63 |

NDS = 1,5-Naphthalindisulfonsäure

**Patentansprüche**

1. Fluorierte 1-Triazolylbutanderivate der allgemeinen Formel I

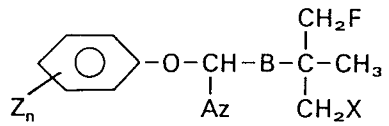

(I)

in welcher

Az für 1,2,4-Triazol-1-yl oder 1,2,4-Triazol-4-yl,

B für die Ketogruppe oder die CH(OH)-Gruppierung,

X für Wasserstoff oder Fluor,

Z für Halogen, Alkyl mit 1 bis 4 Kohlenstoffatomen, Nitro, Cyano, Alkoxycarbonyl mit 1 bis 4 Kohlenstoffatomen im Alkylteil oder für gegebenenfalls durch Halogen substituiertes Phenyl, und

n für 0, 1, 2 oder 3 stehen,

sowie deren Säure- oder Metallsalzkomplexe.

2. Verfahren zur Herstellung von fluorierten 1-Triazolylbutanderivaten der allgemeinen Formel I

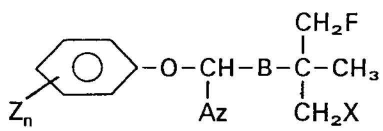

(I)

in welcher

Az für 1,2,4-Triazol-1-yl oder 1,2,4-Triazol-4-yl,

B für die Ketogruppe oder die CH(OH)-Gruppierung,

X für Wasserstoff oder Fluor,

Z für Halogen, Alkyl mit 1 bis 4 Kohlenstoffatomen, Nitro, Cyano, Alkoxycarbonyl mit 1 bis 4 Kohlenstoffatomen im Alkylteil oder für gegebenenfalls durch Halogen substituiertes Phenyl, und

n für 0, 1, 2 oder 3 stehen,

sowie deren Säure- oder Metallsalzkomplexen, dadurch gekennzeichnet, dass man in an sich bekannter Weise Halogenketone der allgemeinen Formel II

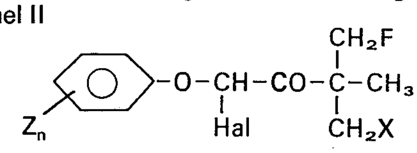

(II)

in welcher

X, Z und n die obenangegebene Bedeutung haben, und

Hal für Halogen, vorzugsweise Chlor oder Brom steht,

mit 1,2,4-Triazol in Gegenwart eines Säurebindemittels und gegebenenfalls in Gegenwart eines Verdünnungsmittels umsetzt und gegebenenfalls noch die so erhaltenen Ketoderivate nach bekannten Methoden in üblicher Weise reduziert und gegebenenfalls noch anschliessend eine Säure oder ein Metallsalz addiert.

3. Fungizide Mittel für den Pflanzenschutz, gekennzeichnet durch einen Gehalt an mindestens einem fluorierten 1-Triazolylbutanderivat der allgemeinen Formel I in Ansprüchen 1 und 2.

4. Verwendung von fluorierten 1-Triazolylbutanderivaten der allgemeinen Formel I in Ansprüchen 1 und 2 zur Bekämpfung von Pilzen im Pflanzenschutz.

5. Verfahren zur Herstellung von fungiziden Mitteln, dadurch gekennzeichnet, dass man fluorierte 1-Triazolylbutanderivate der allgemeinen Formel I in Ansprüchen 1 und 2 mit Streckmitteln und/oder oberflächenaktiven Mitteln vermischt.

**Claims:**

1. Fluorinated 1-triazolylbutane derivatives of the general formula (I):

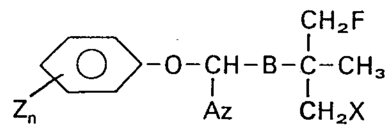

(I)

in which:

Az represents 1,2,4-triazol-1-yl or 1,2,4-triazol-4-yl,

B represents the keto group or the CH(OH)-grouping,

X represents hydrogen or fluorine,

Z represents halogen, alkyl with 1 to 4 carbon atoms, nitro, cyano, alkoxycarbonyl with 1 to 4 carbon atoms in the alkyl part or phenyl which is optionally substituted by halogen, and

n represents 0, 1, 2 or 3,

and acid salt or metal salt complexes thereof.

2. Process for the preparation of fluorinated 1-triazolylbutane derivatives of the general formula (I):

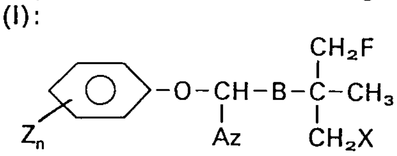

(I)

in which:

Az represents 1,2,4-triazol-1-yl or 1,2,4-triazol-4-yl,

B represents the keto group or the CH(OH)-grouping,

X represents hydrogen or fluorine,

Z represents halogen, alkyl with 1 to 4 carbon atoms, nitro, cyano, alkoxycarbonyl with 1 to 4 carbon atoms in the alkyl part or phenyl which is optionally substituted by halogen, and

n represents 0, 1, 2 or 3,

and acid salt or metal salt complexes thereof, characterized in that halogenoketones of the general formula (II):

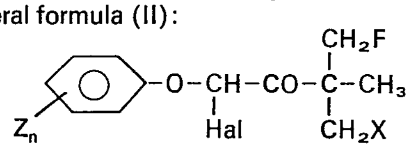

(II)

in which:

X, Z and n have the meaning indicated above, and

Hal represents halogen, preferably chlorine or bromine,

are reacted in a known manner with 1,2,4-triazole

in the presence of an acid-binding agent and if appropriate in the presence of a diluent, and the keto derivatives thus obtained are then optionally reduced by known methods in the customary manner and an acid or a metal salt is then also optionally added on.

3. Fungicidal agents for plant protection, characterized in that they contain at least one fluorinated 1-triazolylbutane derivative of the general formula (I) in claims 1 and 2.

4. Use of fluorinated 1-triazolylbutane derivatives of the general formula (I) in claims 1 and 2 for combating fungi in the protection of plants.

5. Process for the preparation of fungicidal agents, characterized in that fluorinated 1-triazolylbutane derivatives of the general formula (I) in claims 1 and 2 are mixed with extenders and/or surface-active agents.

## Revendications

1. Dérivés fluorés de 1-triazolylbutane de formule générale (I):

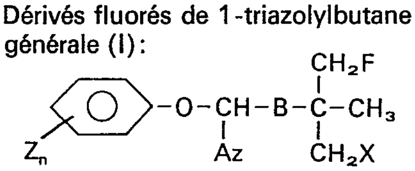

(I)

dans laquelle:

Az représente le reste 1,2,4-triazole-1-yle ou 1,2,4-triazole-4-yle,

B représente le groupe céto ou le groupement $-CH(OH)-$,

X représente l'hydrogène ou le fluor,

Z représente un halogène, un groupe alkyle en $C_1$-$C_4$, nitro, cyano, (alcoxy en $C_1$-$C_4$)-carbonyle ou un groupe phényle éventuellement substitué par un halogène, et

n représente 0, 1, 2 ou 3,

ainsi que leurs complexes d'acides ou de sels métalliques.

2. Procédé pour la fabrication de dérivés fluorés de 1-triazolylbutane de formule générale (I):

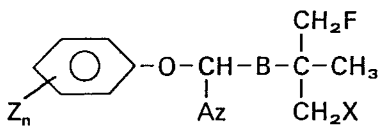

(I)

dans laquelle:

A représente le reste 1,2,4-triazole-1-yle ou 1,2,4-triazole-4-yle,

B représente le groupe céto ou le groupement $-CH(OH)-$,

X représente l'hydrogène ou le fluor,

Z représente un halogène, un groupe alkyle en $C_1$-$C_4$, nitro, cyano, (alcoxy en $C_1$-$C_4$)-carbonyle ou un groupe phényle éventuellement substitué par un halogène, et

n représente 0, 1, 2 ou 3,

ainsi que leurs complexes d'acides ou de sels métalliques, caractérisé en ce que l'on fait réagir, de manière connue, des halogénocétones de formule générale:

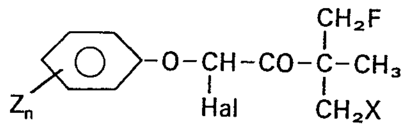

(II)

dans laquelle:

X, Z et n ont la signification indiquée ci-dessus, et

Hal représente un halogène, de préférence le chlore ou le brome,

avec le 1,2,4-triazole en présence d'un accepteur d'acide et, éventuellement, en présence d'un agent diluant; éventuellement, on réduit encore les dérivés céto ainsi obtenus habituellement par des méthodes connues et, éventuellement, on additionne ensuite encore un acide ou un sel métallique.

3. Agents fongicides pour la protection des plantes, caractérisés en ce qu'ils contiennent au moins un dérivé fluoré de 1-triazolylbutane de formule générale (I) selon les revendications 1 et 2.

4. Utilisation des dérivés fluorés de 1-triazolylbutane de formule générale (I) selon les revendications 1 et 2 pour la lutte contre les champignons dans la protection des plantes.

5. Procédé pour la préparation d'agents fongicides, caractérisé en ce que l'on mélange des dérivés fluorés de 1-triazolylbutane de formule générale (I) selon les revendications 1 et 2 avec des agents diluants et/ou des agents tensio-actifs.